# EUROPEAN PATENT SPECIFICATION

(11) **EP 2 367 572 B1**
(45) Date of publication and mention of the grant of the patent: **29.08.2018**
(21) Application number: 09764376.1
(22) Date of filing: 18.11.2009
(51) Int. Cl.: A61K 47/10, A61K 47/14, A61K 47/24, A61K 47/26, A61K 47/44, A61K 31/545

(54) **FORMULATIONS COMPRISING CEFTIOFUR AND BENZYL ALCOHOL**
FORMULIERUNGEN MIT CEFTIOFUR UND BENZYLALKOHOL
FORMULATIONS COMPRENANT DU CEFTIOFUR ET DE L'ALCOOL BENZYLIQUE

(30) Priority: 19.11.2008 US 116031 P; 04.12.2008 US 119764 P
(43) Date of publication of application: 28.09.2011
(73) Proprietor: Merial, Inc., Duluth, GA 30096 (US)
(72) Inventor: RAZZAK, Majid, Auckland (NZ); JOHNSON, Alan, Papatoetoe (NZ); GOSWAMI, Jitendra, Howick (NZ); AWASTHI, Atul, Manukau City (NZ)
(74) Representative: D Young & Co LLP
(86) International application number: PCT/US2009/064978
(87) International publication number: WO 2010/059717

(56) References cited:
- WO-A1-98/41207
- WO-A1-2004/014339
- US-A- 5 736 151
- US-A1- 2004 022 815

## Description

### FIELD OF THE INVENTION

This invention relates to improvements in the field of veterinary remedies and more particularly to improvements in relation to formulations which comprise ceftiofur and benzyl alcohol.

### BACKGROUND OF THE INVENTION

Ceftiofur is a Cephalosporin antibiotic, which is administered to cattle and swine for control of bacterial infections of the respiratory tract. It is used in veterinary medicine as both the sodium salt and the hydrochloride salt. It is administered intramuscularly to cattle and swine. It is also intended to be used as crystalline free acid for intramuscular and subcutaneous administration in cattle and swine. Ceftiofur is poorly absorbed after oral administration while rapidly absorbed after intramuscular administration. The Ceftiofur hydrochloride intramuscular injection is an oily suspension.

Reference is made to U.S. patent application Serial No. 10/211,580 filed August 5, 2002, which published as U.S. Publication No. 20040022815 on February 5, 2004, now abandoned, which describes an oily suspension comprising ceftiofur.

Reference is also made to US 5,736,151 which describes an oil suspension containing a cephalosporin with added water and US 20040022815 which describes an oily suspension of Ceftiofur comprising a resuspendability enhancer. In one embodiment the resuspendability enhancer is selected from polyoxyl hydrogenated vegetable oil, polyoxyl vegetable oil, glycerol, propylene glycol, polyethylene glycols.

A suspension is a particular class or type of dispersion system in which the internal or suspended phase is dispensed uniformly with mechanical agitation through out the external phase, called the suspending medium or vehicle. The internal phase consists of a homogenous or heterogeneous distribution of solid particles having a specific range of sizes, and these particles are maintained uniformly in time throughout the suspending vehicle with the aid of a single, or a particular combination of suspending agents.

The three general classes of pharmaceutical suspensions are orally administered suspensions, externally applied suspensions (topical) and injectable (parenterals) suspensions (Ref: A Martin & P Bustamante, Coarse Dispersion in physical pharmacy 45th edn. Lea and Febiger, Philadelphia, 1993, PP 117-124). Parenteral suspensions are designed for intramuscular, intradermal, intralesional, intraarticular or subcutaneous administration.
Common vehicles for parenteral suspensions include preserved sodium chloride solution or a parenterally acceptable vegetable oil (Ref: J B Partnoff, E M Cohen & M H Henlay, Development of Parenteral and Sterile ophthalmic suspensions - The R&D Approach, Bull. Parenter. Drug Assoc 31: 136-143 (1977)).

The chemical stability of pharmaceutical suspensions is complicated by the factors that affect the physical stability of such suspensions. Since a suspension exists in more than one state (liquid and solid), there are different ways in which the system can undergo either chemical or physical change (Ref: T Higuchi, some physical chemical aspects of suspension formulation, J. Am. Pharm. Assoc. Sci Ed; 47: 657-660 (1958)). Haines & Martin, Hiestand and Econow & Coworkers (Ref: J. Pharm. Sci., 61; 268-272, (1972) and J. Pharm. Sci., 52; 757-762, 1031-1038, (1963)) are generally credited with establishing the structured particle concept or flocculated pharmaceutical suspension.

Flocculation refers to the formation of a loose aggregation of discrete particles held together in a network-like structure either by physical absorption of macromolecules, bridging during chemical interaction, or when the longer range Van der Waals forces of attraction exceeds the shorter range force of repulsion. In agglomeration, a large number of particles are closely bound together as aggregates either in a dry or liquid state. Coagulation or flocculation refers to the massing of particles in a liquid state alone and sometimes in the form of a fluid gel structure.

The main advantages of the stable flocculated systems are as follows. The aggregates tend to break up easily under the application of a small amount of shear stress such as gentle agitation of a bottle or vial or by the flow through a small orifice (hypodermic needle and syringe). In contrast to deflocculated systems, the stable flocculation will settle rapidly and may be easily resuspended even after standing for prolonged time period of storage. The stable flocculation can be produced if required by employing aseptic techniques using vehicle components that are safe for intramuscular injection.

There are several methods of producing flocculated pharmaceutical suspensions. The choice of method depends on the properties of the drug and the class of suspension desired.

One aspect of the present invention is based on Applicants' observation that when a resuspendability enhancer is added to the oily suspension the resuspendability of the suspended particles is improved dramatically, thus giving an improved physical stability to the oily suspension.

Several antibiotic formulations have been used in an attempt to prevent/treat bovine respiratory disease (BRD), which disease is the most significant health problem of the beef industry. Cattle seem to be very susceptible to respiratory disorders. One reason is that the bovine respiratory tract is small relative to body size. Small nostrils limit airflow, increasing breathing effort. A narrow throat passage can easily become dry and irritated, allowing viruses and bacteria to invade.

There are many organisms contributing to the incidence of Bovine Respiratory Disease (BRD). The viral entities include Infectious Bovine Rhino-tracheitis (IBR), Bovine Viral Diarrhea (BVD), Bovine Respiratory Syncytial Virus (BRSV), and Parainfluenza Virus (Ply). The bacteria involved in BRD include *Pasteurella hemolytica, Pasteurella multo-cida, Haemophilus somnus, Mycoplasma* and *Actinomyces pyogenes.*

A complex series of events occurs which is typically associated with BRD. Calves are stressed by weaning, shipping, processing, adverse weather, and over-crowding. This stress compromises the defense mechanisms of the immune system. Viruses invade the nose and lungs because of weakened immune barriers. Viruses damage the epithelium of the upper airways and compromise the effectiveness of the mucociliary apparatus, which sweeps particles (bacteria, dust, mold, and pollen) up and away from the lungs. This lack of ciliary clearance allows overgrowth of normal respiratory inhabitants as well as bacterial pathogens. Secondary bacterial invaders move in, proliferate, and may potentially cause death if the disease is not detected and treated properly.

Prevention of BRD is much more successful and economically feasible than treatment. An ideal processing protocol should include vaccination of these calves two weeks before shipping to allow development of an adequate immune response, and to minimize pre-shipping stress. Calves should receive a booster vaccination once they reach their destination. Vaccination will merely prime an immune response in a healthy immune system. Animals that are immuno-compromised have severely hampered this response.

Identifying the causative agents of BRD can often be difficult and frustrating. Necropsy usually reveals lesions characteristic of secondary bacterial infection, i.e. *Pasteurella hemolytica.* Lesions characteristic of a primary viral agent are often absent due to the lesions of the bacterial agent at the time of death. Viral isolation attempts are usually negative. Antibiotic treatment of these animals prior to death hampers both the ability to isolate bacteria and may affect sensitivity testing.

There remains a need for effective treatments of bovine respiratory disease in cattle. Novel antibiotic formulations with increased efficacy would help address this long felt need. In some instances, anti-inflammatory agents have been successfully combined with antibiotic agents to produce a formulation with improved efficacy against pathogens. One such non-steroidal anti-inflammatory agent, Ketoprofen, may be used as an adjunct to the ceftiofur cephalosporin antibiotic in the treatment of bovine respiratory disease. However, to the best of Applicants' knowledge at the time the instant application was filed, no one had made a suitably effective formulation that comprises both the ceftiofur and Ketoprofen active agents. The availability of one injection product containing both active ingredients, which would reduce the need for multiple injections, is seen as advantageous.

Citation or identification of any document in this application is not an admission that such document is available as prior art to the present invention.

### SUMMARY OF THE INVENTION

The invention provides formulations comprising ceftiofur in combination with benzyl alcohol that are useful for the treating or preventing bovine respiratory disorders. The present invention is based, in part, on Applicants' discovery that addition of benzyl alcohol to an oily ceftiofur HCl formulation resulted in a flocculated suspension that may be resuspended more easily as compared to an oily ceftiofur HCl formulation without benzyl alcohol.

In one embodiment of the first aspect, the present invention relates to a veterinarily or pharmaceutically acceptable formulation comprising: an injectable suspension of an effective amount of ceftiofur or a pharmaceutically acceptable salt thereof; a biocompatible oil vehicle; a wetting or dispersing agent which comprises a lecithin which contains a minimum of 90.0% hydrogenated phosphatidylcholine, a maximum of 4.0% hydrogenated lysophosphatidylcholine, and a maximum of 2% oil or triglycerides; and at least one flocculating agent or resuspendability enhancer, and wherein the at least one flocculating agent or resuspendability enhancer comprises at least benzyl alcohol; wherein the benzyl alcohol is present in an amount of 1% to 5% w/v.

In another embodiment of the first aspect, the ceftiofur is advantageously ceftiofur HCl. In some embodiments, the ceftiofur may be present in an amount of about 0.01% to about 10% w/v. In a more advantageous embodiment, the ceftiofur may be present in an amount of about 5% w/v as the HCl salt. Preferably, the ceftiofur may be present in an amount of about 5.35% w/v as the HCl salt.

The wetting or dispersing agent may be present in an amount of about 0.01% (w/v) to about 1% (w/v) based on the total volume of the formulation. More typically, the wetting or dispersing agent may be present in an amount of about 0.01% (w/v) to about 0.5% (w/v), about 0.01% (w/v) to about 0.1% (w/v), or about 0.05% (w/v) to about 0.2% (w/v). Preferably, the wetting or dispersing agent may be present in an amount of about 0.05% w/v. The wetting or dispersing agent comprises PHOSPHOLIPON 90H. In one embodiment, PHOSPHOLIPON 90H may be present in an amount of about 0.01% to about 0.10% w/v, and even more advantageously, about 0.05% w/v.

In yet another embodiment of the first aspect, the flocculating agent or resuspendability enhancer may further comprise propylene glycol. The propylene glycol is typically present in an amount of about 0.01% (w/v) to about 5% (w/v). More typically, the propylene glycol may be present in an amount of about 0.01% to about 1% (w/v) or about 0.01% to about 0.5% (w/v). Advantageously, the propylene glycol may be present in an amount of about 0.25% w/v.

The benzyl alcohol is present in an amount of 1% to 5% w/v. In particular, the benzyl alcohol may be present in an amount of 1%, about 2% or about 3% w/v.

In another embodiment, the formulations of the invention may comprise chlorobutanol. In some embodiments, the chlorobutanol may be present in an amount of about 0.01% to about 10% w/v. More typically, the formulations may include chlorobutanol in an amount of about 0.1% to about 5%, about 0.1% to about 1% w/v. Preferably, the formulations may contain chlorobutanol in an amount of about 0.5% w/v.

In another advantageous embodiment of the first aspect, the biocompatible oil vehicle may comprise cottonseed oil.

In another advantageous embodiment of the first aspect, the invention pertains to a formulation which comprises (a) ceftiofur HCl, (b) benzyl alcohol (c) a lecithin which contains a minimum of 90.0% hydrogenated phosphatidylcholine, a maximum of 4.0% hydrogenated lysophosphatidylcholine, and a maximum of 2% oil or triglycerides, (d) sorbitan monooleate, (e) propylene glycol and (f) cottonseed oil.

In a particularly advantageous embodiment of the first aspect, the invention relates to a formulation wherein (a) ceftiofur HCl may be present in an amount of about 5.35% w/v, (b) benzyl alcohol may be present in an amount of about 1% to about 3% w/v, (c) a lecithin which contains a minimum of 90.0% hydrogenated phosphatidylcholine, a maximum of 4.0% hydrogenated lysophosphatidylcholine, and a maximum of 2% oil or triglycerides may be present in an amount of about 0.05% w/v, (d) sorbitan monooleate may be present in an amount of about 0.15% w/v, (e) propylene glycol may be present in an amount of about 0.25% w/v and (f) cottonseed oil may be present in an amount of up to about 100% w/v.

Also described herein (without being part of the invention) is a method of improving resuspendability of an oil based formulation which comprises adding benzyl alcohol to the oil based formulation, thereby improving resuspendability. Advantageously, the oil based formulation may be a ceftiofur formulation.

Also described herein (without being part of the invention) is the Applicant's discovery that a formulation comprising an effective amount of ceftiofur and ketoprofen is effective in cattle for the treatment and/or prevention of bovine respiratory disease (BRD).

Described herein (without being part of the invention) is therefore a formulation which may comprise (a) ceftiofur and (b) ketoprofen.

Also described (without being part of the invention) is a formulation which may comprise (a) ceftiofur, (b) ketoprofen, (c) wetting and/or dispersing agent(s), (d) a preservative, (e) a flocculating agent or resuspendability enhancer and (f) a biocompatible oil vehicle.

The ceftiofur may be ceftiofur HCl. The ceftiofur may be present in an amount of about 0.01% w/v to about 10% w/v, about 1% w/v to about 8% w/v, advantageously about 2% w/v to about 7% w/v, and more advantageously, about 5% w/v. The ketoprofen may be present in an amount of about 0.01% w/v to about 30% w/v, about 5% w/v to about 25% w/v, advantageously about 10% w/v to about 20% w/v, and more advantageously, about 15% w/v.

The wetting or dispersing agent may comprise a hydrogenated phosphatidylcholine, a hydrogenated lysophosphatidylcholine, a mono-diglyceride, propylene glycol, a triglyceride or combinations thereof. One of the wetting or dispersing agents may comprise sorbitan monooleate. The wetting or dispersing agents may be present in amounts according to those described above for the invention.

The preservative may comprise benzyl alcohol or chlorobutanol.

The flocculating agent or resuspendability enhancer may comprise propylene glycol.

The biocompatible oil vehicle may comprise an ester of caprylic acid, an ester of capric fatty acids, propylene glycol or a combination thereof. Advantageously, the biocompatible oil may be MIGLYOL 840.

The benzyl alcohol, chlorobutanol, propylene glycol, caprylic acid or capric fatty acid esters and MIGLYOL 840 may be present in the formulation in the same amounts as described above for the invention.

A formulation (which is not part of the invention) may comprise: (a) ceftiofur, (b) ketoprofen, (c) PHOSPHOLIPON 90H, (d) benzyl alcohol or chlorobutanol, (e) sorbitan monooleate, (f) propylene glycol and (g) MIGLYOL 840.

Also described is a formulation (which is not part of the invention) wherein (a) ceftiofur may be present in an amount of about 5% w/v, (b) ketoprofen may be present in an amount of about 15% w/v, (c) PHOSPHOLIPON 90H may be present in an amount of about 0.05% w/v, (d) benzyl alcohol may be present in an amount of about 1% w/v and/or chlorobutanol may be present in an amount of about 0.5% w/v, (e) sorbitan monooleate may be present in an amount of about 0.15% w/v, (f) propylene glycol may be present in an amount of about 0.25% w/v and (g) MIGLYOL 840 may be present in an amount of up to about 100% w/v.

In a second aspect, the present invention also relates to a formulation of the invention for use in preventing or treating a respiratory disorder or disease in an animal.

In one embodiment of this second aspect of the present invention, the formulation is formulated to be administered by injection.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of' have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings, in which:

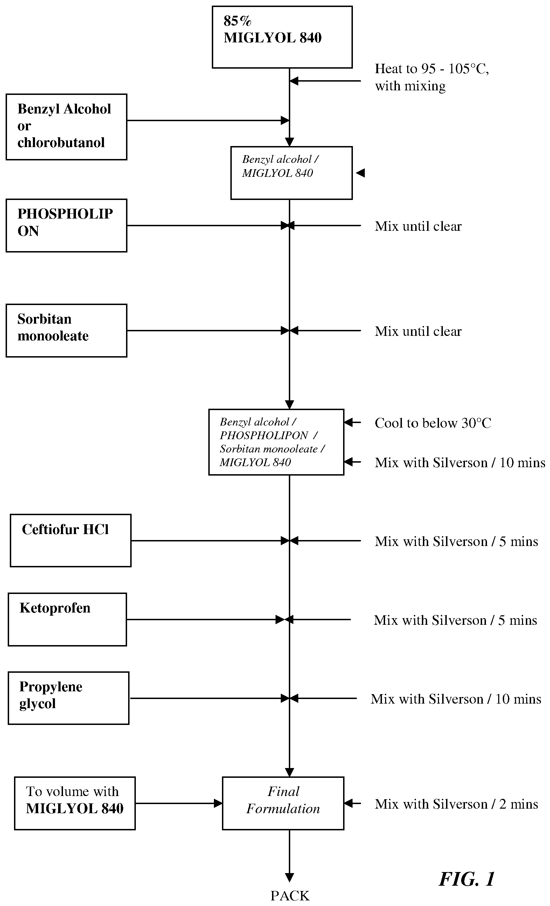

FIG. 1 depicts a schematic diagram of a manufacturing process (which is not part of the invention).

### DETAILED DESCRIPTION

The instant invention provides formulations comprising ceftiofur in combination with benzyl alcohol. Also described herein are formulations (not according to the invention) comprising ceftiofur in combination with ketoprofen.
The formulations comprising ceftiofur or ceftiofur and ketoprofen in combination with benzyl alcohol have improved flocculation properties and are substantially easier to resuspend. Formulations comprising ceftiofur and ketoprofen provide improved efficacy in treating and/or preventing bovine respiratory disorders, particularly, bovine respiratory disease (BRD).

Ceftiofur is a cephalosporin antibiotic, which may be administered, for example, by intramuscular or subcutaneous injection to cattle and swine for control of bacterial infections of the respiratory tract. Ceftiofur may be administered as a neutral compound or as a pharmaceutically or veterinarily acceptable salt. In a preferred embodiment, the formulations of the invention comprise ceftiofur HCl.

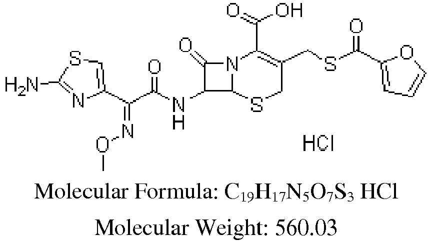

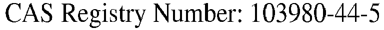

In one embodiment, the dosage of ceftiofur administered to cattle by intramuscular injection may be about 0.1 mg/kg to about 5 mg/kg according to the weight of the animal. In certain other embodiments, the dosage of ceftiofur administered to cattle by this mode of administration may be about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1.0 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg or about 2.2 mg/kg once daily for three days.

Ceftiofur HCl is commercially available and may be supplied by various suppliers including, for example, Orchid Chemical or Pharmaceuticals Ltd or Hisun Pharmaceutical Co. Ltd., among others. Pharmaceutically acceptable salts of ceftiofur include, but are not limited to, the sodium, hydrochloride or hydrobromide salts.
Ketoprofen is a non-steroidal anti-inflammatory agent which may be used as an adjunct to the ceftiofur cephalosporin antibiotic in the treatment of bovine respiratory disease. The availability of one injection product containing both active ingredients, which would reduce the need for multiple injections, is seen as advantageous.

Ketoprofen may be administered, for example, by intravenous or intramuscular injection in cattle.

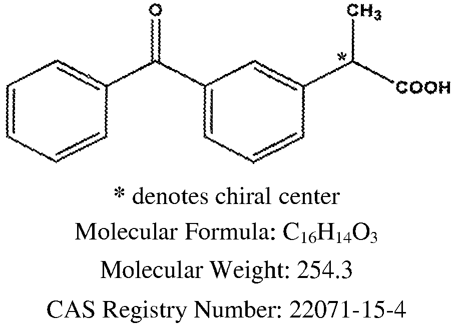

This specification contemplates all pharmaceutically or veterinary acceptable acid or base salt forms of the ceftiofur and ketoprofen. The term "acid" contemplates all pharmaceutically or veterinary acceptable inorganic or organic acids. Inorganic acids include mineral acids such as hydrohalic acids, such as hydrobromic and hydrochloric acids, sulfuric acids, phosphoric acids and nitric acids. Organic acids include all pharmaceutically or veterinary acceptable aliphatic, alicyclic and aromatic carboxylic acids, dicarboxylic acids tricarboxylic acids and fatty acids. Preferred acids are straight chain or branched, saturated or unsaturated C₁-C₂₀ aliphatic carboxylic acids, which are optionally substituted by halogen or by hydroxyl groups, or C₆-C₁₂ aromatic carboxylic acids. Examples of such acids are carbonic acid, formic acid, fumaric acid, acetic acid, propionic acid, isopropionic acid, valeric acid, α-hydroxy acids, such as glycolic acid and lactic acid, chloroacetic acid, benzoic acid, methane sulfonic acid, and salicylic acid. Examples of dicarboxylic acids include oxalic acid, malic acid, succinic acid, tartaric acid and maleic acid. An example of a tricarboxylic acid is citric acid. Fatty acids include all pharmaceutically or veterinary acceptable saturated or unsaturated aliphatic or aromatic carboxylic acids having 4 to 24 carbon atoms. Examples include butyric acid, isobutyric acid, sec-butyric acid, lauric acid, palmitic acid, stearic acid, oleic acid, linoleic acid, linolenic acid, and phenylsteric acid. Other acids include gluconic acid, glycoheptonic acid and lactobionic acid.

The term "base" contemplates all pharmaceutically or veterinary acceptable inorganic or organic bases. Such bases include, for example, the alkali metal and alkaline earth metal salts, such as the lithium, sodium, potassium, magnesium or calcium salts. Organic bases include the common hydrocarbyl and heterocyclic amine salts, which include, for example, the morpholine and piperidine salts.
The ester and amide derivatives of these compounds, where applicable, are also contemplated.

In various embodiments, the ceftiofur may be present in the formulation in an amount of about 0.01% w/v to about 10% w/v, about 1% w/v to about 8% w/v, advantageously about 2% w/v to about 7% w/v, and more advantageously, about 5% w/v.

In the described formulations (not according to the invention), the dosage of ketoprofen administered to cattle by intramuscular injection is about 0.1 mg/kg to about 10 mg/kg according to the weight of the animal. Or the dosage of ketoprofen administered to cattle by intramuscular injection may be about 1.0 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg, about 2.2 mg/kg, about 2.3 mg/kg, about 2.4 mg/kg, about 2.5 mg/kg, about 2.6 mg/kg, about 2.7 mg/kg, about 2.8 mg/kg, about 2.9 mg/kg, about 3.0 mg/kg, about 3.1mg/kg, about 3.2mg/kg , about 3.3mg/kg , about 3.4mg/kg , about 3.5mg/kg , about 3.6mg/kg , about 3.7mg/kg , about 3.8mg/kg , about 3.9mg/kg , about 4.0mg/kg, about 4.1mg/kg, about 4.2mg/kg or about 4.3mg/kg once daily for three days.

Ketoprofen is commercially available, and may be supplied, for example, by Zhejang Jiuzhou Pharmaceutical Company, Jinan Haohua Industry Co., Ltd., King Tang Chemical Group Industry Co., Ltd, Greatvista Chemicals, Boehringer Ingelheim- Biopharmaceuticals, LKT Laboratories, Sigma-Aldrich, Difco Microbiology Products, Glaxosmithkline Pharmaceuticals S.A., or other sources.

In a first aspect, the present invention provides improved oil-based formulations comprising ceftiofur and benzyl alcohol that exhibit superior resuspendability compared to formulations of the prior art.

In one embodiment of the first aspect, the present invention relates to a veterinarily or pharmaceutically acceptable formulation comprising:
an injectable suspension of an effective amount of ceftiofur, or a pharmaceutically acceptable salt thereof;
a biocompatible oil vehicle;
a wetting or dispersing agent which comprises a lecithin which contains a minimum of 90.0% hydrogenated phosphatidylcholine, a maximum of 4.0% hydrogenated lysophosphatidylcholine, and a maximum of 2% oil or triglycerides; and
   at least one flocculating agent or resuspendability enhancer, and wherein the at least one flocculating agent or resuspendability enhancer comprises at least benzyl alcohol; wherein the benzyl alcohol is present in an amount of 1% to 5% w/v.

Benzyl alcohol is a useful solvent due to its polarity, low toxicity and low vapor pressure and is commonly added to intravenous medication solutions as a preservative due to its bacteriostatic and antipruritic properties.

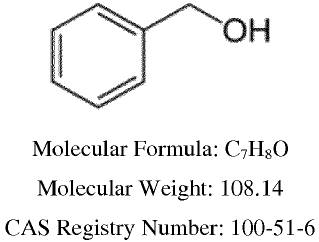

The present invention also contemplates other aromatic alcohols in addition to benzyl alcohol. Generally, aromatic alcohol compounds contain a hydroxyl group bonded to an aromatic moiety. In some cases, the hydroxyl group may be bonded directly to the aromatic group including, but not limited to, phenyl or substituted phenyl groups, naphthyl and substituted naphthyl groups, and the like. In other compounds the hydroxyl group may be bonded to the aromatic ring by a linker moiety such as an alkylene group. Suitable aromatic alcohols of both types are encompassed by the present invention. Even though phenol is technically an aromatic alcohol, it is not contemplated for the present invention.

Ceftiofur hydrochloride oily suspension is a flocculated suspension. In aqueous suspensions, flocculation occurs when the zeta potential is reduced and attractive forces exceed repulsive forces. Benzyl alcohol may be involved in the flocculation process (US patent 3,457,348). This patent relates to an interaction of hydroxy preservatives with nonionic surfactants and is silent to the involvement of benzyl alcohol in the flocculation process in oil based formulations.

The formulations of the present invention that comprise benzyl alcohol exhibit improved flocculation and a reduced rate of sedimentation. Factors known to affect sedimentation rate are:
- Particle size and shape
- Density difference between dispersed phase and dispersion media
- Viscosity of the dispersion media

For benzyl alcohol to reduce the rate of sedimentation, it must alter the floc in some way either by the size of the floc or the porosity of the floc.

Advantageously, the benzyl alcohol is present in an amount of 1% to 5% (w/v). In particular, the benzyl alcohol may be present in an amount of 1%, about 2% or about 3% w/v in the formulations of the invention.

The wetting or dispersing agent may be present in an amount of about 0.01% (w/v) to about 1% (w/v) based on the total volume of the formulation. More typically, the wetting or dispersing agent may be present in an amount of about 0.01% (w/v) to about 0.5% (w/v), about 0.01% (w/v) to about 0.1% (w/v), or about 0.05% (w/v) to about 0.2% (w/v). Preferably, the wetting or dispersing agent may be present in an amount of about 0.05% w/v. The wetting or dispersing agent comprises PHOSPHOLIPON 90H, in the invention.

The lecithin PHOSPHOLIPON 90H may be supplied by Phospholipid GmbH and contains a minimum of 90.0% hydrogenated phosphatidylcholine, a maximum of 4.0% hydrogenated lysophosphatidylcholine, and a maximum of 2% oil or triglycerides.

In yet another embodiment, the formulations may include a further flocculating agent or resuspendability enhancer that comprises propylene glycol. The propylene glycol is typically present in an amount of about 0.01% (w/v) to about 5% (w/v). More typically, the propylene glycol may be present in an amount of about 0.01% to about 1% (w/v) or about 0.01% to about 0.5% (w/v). Advantageously, the propylene glycol may be present in an amount of about 0.25% w/v

Other flocculating agents or resuspendability enhancers including, but not limited to, polyoxyl hydrogenated castor oil, polyoxyl castor oil, glycerol, polyoxyl hydrogenated vegetable oil, polyoxyl vegetable oil, glycerol, polyethylene glycol, alcohols and the like are also contemplated for the present invention.

In one embodiment, the biocompatible oil vehicle may comprise an ester of caprylic acid, an ester of capric fatty acids, propylene glycol or a combination thereof. Advantageously, the biocompatible oil may be MIGLYOL 840.

MIGLYOL 840 is a clear, neutral oil consisting of the esters of caprylic and capric fatty acids (obtained from coconut and palm kernel oils) and propylene glycol. It is a particularly low viscosity oil with a specification of 9 to 12 mPas at 20°C and obtained from Sasol GmbH.

In another advantageous embodiment, the biocompatible oil vehicle may comprise cottonseed oil.

Other biocompatible oils which are contemplated by the present invention include, but are not limited to, monoglyceride, diglyceride, triglyceride medium chain succinic acid triglyceride, corn oil, cottonseed oil, olive oil, sesame oil, soybean oil, safflower oil, coconut oil, sunflower oil, palm oil, peanut oil, corn oil, an ester of caprylic acid, or an ester of capric fatty acids, propylene glycol, or combinations thereof.

In another embodiment of the first aspect of the invention, the formulations may comprise chlorobutanol. In some embodiments, the chlorobutanol may be present in an amount of about 0.01% to about 10% w/v. More typically, the formulations may include chlorobutanol in an amount of about 0.1% to about 5%, about 0.1% to about 1% w/v. Preferably, the formulations may contain chlorobutanol in an amount of about 0.5% w/v.

In another advantageous embodiment of the first aspect, the invention pertains to a formulation which comprises: (a) ceftiofur HCl, (b) benzyl alcohol (c) a lecithin which contains a minimum of 90.0% hydrogenated phosphatidylcholine, a maximum of 4.0% hydrogenated lysophosphatidylcholine, and a maximum of 2% oil or triglycerides, (d) sorbitan monooleate, (e) propylene glycol and (f) cottonseed oil.

In a particularly advantageous embodiment of the first aspect, the invention relates to a formulation wherein (a) ceftiofur HCl may be present in an amount of about 5.35% w/v, (b) benzyl alcohol may be present in an amount of about 1% to about 3% w/v, (c) a lecithin which contains a minimum of 90.0% hydrogenated phosphatidylcholine, a maximum of 4.0% hydrogenated lysophosphatidylcholine, and a maximum of 2% oil or triglycerides may be present in an amount of about 0.05% w/v, (d) sorbitan monooleate may be present in an amount of about 0.15% w/v, (e) propylene glycol may be present in an amount of about 0.25% w/v and (f) cottonseed oil may be present in an amount to complement the volume of the formulation to 100%.

Also described herein (not according to the invention) is a method of improving resuspendability of an oil based formulation comprising adding benzyl alcohol to the formulation, thereby improving resuspendability. Advantageously, the oil based formulation may be a ceftiofur formulation.

The method may comprise adding the benzyl alcohol to the formulation in an amount of about 0.05% to about 10% w/v, more advantageously about 0.5% to about 5% (w/v). Or the method comprises adding benzyl alcohol to the formulation in an amount of about 1%, about 2% or about 3% w/v.

Also described herein (not according to the invention) is a formulation comprising a combination of ceftiofur and ketoprofen that is effective in cattle for the treatment and/or prevention of bovine respiratory disease (BRD). It has been surprisingly found that certain combinations of ceftiofur and ketoprofen provide superior efficacy in the treatment and/or prevention of BRD.

The described formulation (not according to the invention) may comprise (a) ceftiofur, (b) ketoprofen, (c) wetting and/or dispersing agent(s), (d) a preservative, (e) a flocculating agent or resuspendability enhancer and (f) a biocompatible oil vehicle.

The ceftiofur may be ceftiofur HCl.

The ceftiofur may be present in an amount of about 0.01% w/v to about 10% w/v, about 1% w/v to about 8% w/v, advantageously about 2% w/v to about 7% w/v, and more advantageously, about 5% w/v. The ketoprofen may be present in an amount of about 0.01% w/v to about 30% w/v, about 5% w/v to about 25% w/v, advantageously about 10% w/v to about 20% w/v, and more advantageously, about 15% w/v.

The wetting or dispersing agent in the formulations may comprise the same wetting or dispersing agents used in formulations comprising ceftiofur and benzyl alcohol described above for the first aspect of the invention. The wetting or dispersing agents may be used in the same amounts as described above for the first aspect of the invention. The formulations may comprise a hydrogenated phosphatidylcholine, a hydrogenated lysophosphatidylcholine, a biocompatible oil, triglycerides, PHOSPHOLIPON 90H, sorbitan monooleate, or combinations thereof.

The wetting or dispersing agent may be present in an amount of about 0.01% (w/v) to about 1% (w/v) based on the total volume of the formulation. More typically, the wetting or dispersing agent may be present in an amount of about 0.01% (w/v) to about 0.5% (w/v), about 0.01% (w/v) to about 0.1% (w/v), or about 0.05% (w/v) to about 0.2% (w/v). Preferably, the wetting or dispersing agent may be present in an amount of about 0.05% w/v.

One of the wetting or dispersing agents may comprise sorbitan monooleate, which may be present in an amount of about 0.01% w/v to about 1% w/v, about 0.01% to about 0.3%, and more advantageously about 0.15% w/v.

The formulation may comprise benzyl alcohol or chlorobutanol. Advantageously, the benzyl alcohol may be present in an amount of about 0.01% w/v to about 10% w/v, or about 0.5% to about 5% w/v. More typically, the benzyl alcohol may be present in an amount of about 1%, about 2%, or about 3% w/v.

The chlorobutanol may be present in an amount of about 0.01% to about 10% w/v. More typically, the formulations may include chlorobutanol in an amount of about 0.1% to about 5%, about 0.1% to about 1% w/v. Preferably, the formulations may contain chlorobutanol in an amount of about 0.5% w/v.

The flocculating agent or resuspendability enhancer may comprise propylene glycol. Advantageously, the propylene glycol may be present in an amount of about 0.01% w/v to about 2% w/v, about 0.05% w/v to about 1% w/v, and more advantageously about 0.25% w/v.

The biocompatible oil vehicle may comprise an ester of caprylic acid, an ester of capric fatty acids, propylene glycol or a combination thereof. Advantageously, the biocompatible oil may be MIGLYOL 840.

The formulations may comprise other components that are described above for formulations comprising ceftiofur and benzyl alcohol of the first aspect of the invention.

The formulation may comprise: (a) ceftiofur, (b) ketoprofen, (c) PHOSPHOLIPON 90H, (d) benzyl alcohol or chlorobutanol, (e) sorbitan monooleate, (f) propylene glycol and (g) MIGLYOL 840.

Described herein (not according to the invention) is a formulation wherein (a) ceftiofur may be present in an amount of about 5% w/v, (b) ketoprofen may be present in an amount of about 15% w/v, (c) PHOSPHOLIPON 90H may be present in an amount of about 0.05% w/v, (d) benzyl alcohol may be present in an amount of about 1% w/v or chlorobutanol may be present in an amount of about 0.5% w/v, (e) sorbitan monooleate may be present in an amount of about 0.15% w/v, (f) propylene glycol may be present in amount of about 0.25% w/v and (g) MIGLYOL 840 may be present in an amount of up to about 100% w/v.

In a second aspect, the present invention provides a formulation of the first aspect of the invention for use in treating or preventing a respiratory disorder or disease in an animal, including livestock animals. The formulations of the invention are formulated for injectable administration.

As the finished product may be an injectable product in a multi-use vial, it needs to be both sterile and capable of maintaining its sterility with multiple challenges. As an oily suspension the alternative techniques to produce a sterile product are either by aseptic manufacture or gamma irradiation.

The present invention also contemplates administering the formulations using a needlefree injector such as PIGJET®, AVIJET®, DERMOJET® or BIOJECTOR® (Bioject, Oregon, USA). A person of ordinary skill in the art is able to adjust the specifications of the injector as required with regard to factors such as the species of the animal to be treated; the age and weight of the animal, and the like without undue experimentation.

The composition containing the ceftiofur of the invention may be administered continuously, for treatment or prophylaxis, by known methods. In one embodiment, a dose of from about 0.001 to about 50 mg per kg of body weight given as a single dose or in divided doses for a period of from 1 to 5 days will be satisfactory but, of course, there can be instances where higher or lower dosage ranges are indicated, and such are within the scope of this invention. It is well within the routine skill of the practitioner to determine a particular dosing regimen.

In one embodiment, the treatment is carried out so as to administer to the animal, on a single occasion, a dose containing between about 0.001 and about 100 mg/kg of the ceftiofur or between about 0.1 and about 200 µg/kg or about 100 µg/kg of the compound.

The composition containing the ceftiofur and ketoprofen described herein may be administered continuously, for treatment or prophylaxis, by known methods. Generally, a dose of from about 1 to about 20 mg of ceftiofur and of ketoprofen per kg of body weight given as a single dose or in divided doses for a period of from 1 to 5 days will be satisfactory but, of course, there can be instances where higher or lower dosage ranges are indicated.

In one embodiment, the dosage of ceftiofur to be administered to cattle by intramuscular injection may be about 0.1 mg/kg to about 5 mg/kg according to the weight of the animal. In certain other embodiments, the dosage of ceftiofur to be administered to cattle by this mode of administration may be about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1.0 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg or about 2.2 mg/kg once daily for three days.

The dosage of ketoprofen which may be administered to cattle by intramuscular injection is about 0.1 mg/kg to about 10 mg/kg according to the weight of the animal. Or the dosage of ketoprofen which may be administered to cattle by intramuscular injection may be about 1.0 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg/kg, about 2.2 mg/kg, about 2.3 mg/kg, about 2.4 mg/kg, about 2.5 mg/kg, about 2.6 mg/kg, about 2.7 mg/kg, about 2.8 mg/kg, about 2.9 mg/kg, about 3.0 mg/kg, about 3.1 mg/kg, about 3.2mg/kg , about 3.3mg/kg , about 3.4 mg/kg , about 3.5 mg/kg , about 3.6 mg/kg , about 3.7 mg/kg , about 3.8 mg/kg , about 3.9 mg/kg , about 4.0 mg/kg, about 4.1 mg/kg, about 4.2 mg/kg or about 4.3 mg/kg once daily for three days.

On a single occasion, a dose containing between about 1 and about 20 mg/kg of ceftiofur and of ketoprofen may be administered.

During formulation development, investigation of the physical characteristics of the suspensions produced tended to be carried out with the formulations stored in either 100 mL clear glass measuring cylinders or 100 mL glass vials (either clear or amber), or in many cases both. The proposed packaging format may be 100 mL clear, type I glass vials with stoppers. The present description also contemplates the administration of the ceftiofur and ketoprofen in separate compartments, to be admixed upon administration to the animal.

The invention will now be further described by way of the following non-limiting examples.

### EXAMPLES

### EXAMPLE 1

### Effect of benzyl alcohol on the physical properties of ceftiofur HCl oily suspensions.

**TABLE 1**

| Lab batches prepared with 1% benzyl alcohol and without benzyl alcohol. Batches A, C and E are not according to the invention. | | | | | | | | |
|---|---|---|---|---|---|---|---|---|
| Batch | A | B | C | D | E | F | G | H |
| | % w/v | % w/v | % w/v | % w/v | % w/v | % w/v | % w/v | % w/v |
| Ceftiofur HCl | 5.35 | 5.35 | 5.35 | 5.35 | 5.35 | 5.35 | 5.35 | 5.35 |
| PHOSPHOLIPON 90H | 0.05 | 0.05 | 0.05 | 0.05 | 0.05 | 0.05 | 0.05 | 0.05 |
| Sorbitan monooleate | 0.15 | 0.15 | 0.15 | 0.15 | 0.15 | 0.15 | 0.15 | 0.15 |
| Propylene Glycol | 0.25 | 0.25 | 0.25 | 0.25 | 0.25 | 0.25 | 0.25 | 0.25 |
| Benzyl alcohol | none | 1.0 | none | 1.0 | none | 1.0 | 2.0 | 3.0 |
| Cottonseed oil | to volume | to volume | to volume | to volume | to volume | to volume | to volume | to volume |

An observation was made that separation on standing in a rectangular 100mL laboratory bottle was greater for the formulation without benzyl alcohol than for the batch with benzyl alcohol. This result was surprising and unexpected.

Initial observations revealed separation at 4 days in a 100 mL rectangular laboratory bottle. The sedimentation volume, F, is the ratio of the equilibrium volume of the sediment, Vᵤ, to the total volume of the suspension, V₀. Thus, F = Vᵤ / V₀. As the volume of suspension that appears occupied by the sediment increases, the value of F, which normally ranges from nearly 0 to 1, increases. In the system where F = 0.75, for example, 75% of the total volume in the container is apparently occupied by the loose, porous flocs forming the sediment. When F = 1, no sediment is apparent even though the system is flocculated. This is an ideal suspension because, under these conditions, no sedimentation occurs. The Sedimentation Volume (%) is equal to: [(Volume of Sediment x 100) / (Total Volume)].

**TABLE 2**

| Sedimentation at 4 days | |
|---|---|
| Batch A (No benzyl alcohol) | 54% sedimentation |
| Batch B (1%) benzyl alcohol) | 83% sedimentation |
| Previous batches (containing 1% benzyl alcohol) | 76-84% typical |

Testing was conducted with 0% versus 1% Benzyl alcohol. The sedimentation volume (%) on standing in a 100 mL measuring cylinder is summarized in TABLE 3.

**TABLE 3**

| Sedimentation Volume (%) = [(Volume of Sediment x 100) / (Total Volume)] | | |
|---|---|---|
| | Batch A | Batch B |
| Benzyl alcohol | 0% | 1% |
| Initial | 100% | 100% |
| 1 day | NR | NR |
| 2 days | 95% | 97% |
| 3 days | NR | NR |
| 4 days | 69% | 73% |
| 6 days | NR | NR |
| 7 days | 55% | 72% |
| 17 days | 30% | 54% |
| 22 days | 28% | 51% |
| 31 days | 26% | 48% |
| 37 days | 25% | 46% |
| 43 days | 245 | 45% |
| 56 days | 23% | 44% |

| | Batch A | Batch B |
|---|---|---|
| 180 days | 22% | 42% |

| | | |
|---|---|---|
| NR = not recorded | | |

The sedimentation volume on sanding in a 100 mL round injection vial is summarized in TABLE 4. TABLE 5 depicts flow rate, as measured by determining the flow rate through an orifice at the base of a Ford cup No. 4 loaded with a specified volume of suspension. The results of TABLE 5 demonstrate that the presence of the benzyl alcohol has no effect on the viscosity of the suspension.

**TABLE 4**

| Sedimentation Volume (%) = [(Volume of Sediment x 100) / (Total Volume)]: | | |
|---|---|---|
| | Batch C | Batch D |
| Benzyl alcohol | 0% | 1% |
| Initial | 100% | 100% |
| 1 day | 90% | 95% |
| 2 days | 79% | NR |
| 4 days | NR | 90% |
| 5 days | 49% | 86% |
| 7 days | 41% | 83% |

| | | |
|---|---|---|
| NR = not recorded | | |

**TABLE 5 Flow rate in a Ford cup No. 4**

| | Flow rate | % Benzyl alcohol |
|---|---|---|
| Batch A | 26 seconds | 0% |
| Batch B | 27 seconds | 1% |
| Other lab batches | 27-31 seconds | 1% |

A centrifuge-resuspension test was conducted as follows: 8 mL was introduced into a 10 mL round bottom tube. The tube was placed in the centrifuge at 1000 rpm for 15 minutes. The following measurements were recorded: volume of sediment and the time to resuspend the product (leaving no trace of residue on the bottom of the tube). After sedimentation, resuspension was performed in 5 second intervals, holding the tube horizontally and allowing 3 back and forward movements per second.

**TABLE 6**

| Centrifuge-resuspension test | | |
|---|---|---|
| | Batch A | Batch B |
| Benzyl alcohol | 0% | 1% |
| Sedimentation | 92% | 80% |
| Resuspension time | 35 seconds | 15 seconds |

Syringeability and injectability tests were also conducted. 2 mL of sample was drawn from an inverted injection vial through an 18 gauge needle into a 3 mL syringe. The contents of the syringe were then discharged. Syringeability and injectability were acceptable. The test was repeated with a glass syringe. Both syringeability and injectability improved with glass.

To summarize, sedimentation on standing is faster for the formulation without Benzyl alcohol than for the formulation containing 1% Benzyl alcohol. Centrifuge and resuspension testing showed resuspension time was quicker for a formulation containing 1% Benzyl alcohol. Ford cup flow rate is not significantly affected by the benzyl alcohol. There was no variation in syringeability and injectability between formulation batch A and batch B.

Batches were then manufactured with 0, 1.0, 2.0 and 3.0% Benzyl alcohol. The sedimentation volume on standing in a 100 mL measuring cylinder is summarized in TABLE 7.

**TABLE 7**

| Sedimentation Volume (%) = [(Volume of Sediment x 100) / (Total Volume)] | | | | |
|---|---|---|---|---|
| | Batch E | Batch F | Batch G | Batch H |
| Benzyl alcohol | 0% | 1% | 2% | 3% |
| 1 day | 97% | 94% | 99% | 99% |
| 2 days | 90% | 86% | 97% | 98% |
| 3 days | 82% | 81% | 96% | 96% |
| 4 days | 73% | 75% | 95% | 95% |
| 5 days | 64% | 69% | 93% | 94% |
| 6 days | 56% | 64% | 92% | 94% |
| 7 days | 42% | 61% | 91% | 93% |

### EXAMPLE 2

### Comparison of benzyl alcohol-containing ceftiofur batches with EXCENEL® RTU

Ceftiofur formulations were manufactured with 0, 1.0, 2.0 and 3.0% benzyl alcohol (batches E-H, respectively), and their physical properties were compared to those of EXCENEL® RTU Sterile Suspension manufactured by Pfizer (ceftiofur hydrochloride), which contains no benzyl alcohol. The sedimentation volumes on standing in a 100 mL graduated cylinder were measured. Sedimentation Volume (%) = [(Volume of Sediment x 100) / (Total Volume)] and the results are summarized in TABLE 8.

**TABLE 8**

| Sedimentation Volume (%) = (Volume of Sediment x 100)/Total Volume) | | | | | |
|---|---|---|---|---|---|
| | Batch E | Batch F | Batch G | Batch H | EXCENEL® RTU |
| Benzyl | 0% | 1% | 2% | 3% | 0% |
| 1 day | 92% | 93% | 99% | 99% | 90% |
| 2 days | 78% | 83% | 83% | 85% | 75% |
| 3 days | 58% | 82% | 82% | 82% | NR |
| 4 days | 46% | 79% | 79% | 78% | 49% |
| 5 days | 42% | 75% | 75% | 75% | 39% |
| 6 days | NR | NR | NR | NR | NR |
| 7 days | NR | NR | NR | NR | 36% |

| | | | | | |
|---|---|---|---|---|---|
| NR = not recorded | | | | | |

The viscosity in a Brookfield LV 2 at 100 rpm was measured and the results are summarized in TABLE 9.

**TABLE 9**

| Viscosity | | | | |
|---|---|---|---|---|
| | Batch E | Batch F | Batch G | Batch H |
| Benzyl alcohol | 0% | 1% | 2% | 3% |
| | 129 cps | 136 cps | 138 cps | 142 cps |

The viscosity of Batch E is 129 mPas (cps), the viscosity of Batch F is 136 mPas (cps), the viscosity of Batch G is 138 mPas (cps), and the viscosity of Batch H is 142 mPas (cps). A centrifuge-resuspension test was conducted as follows. 8 mL was introduced into a 10 mL round bottom tube. The tube was placed in the centrifuge at 1000 rpm for 15 minutes. The following measurements were recorded: volume of sediment and time to resuspend the formulation (leaving no trace of residue on the bottom of the tube). Following sedimentation, resuspension was performed in 5 second intervals, holding the tube horizontally and allowing 3 back and forward movements per second. The results are summarized in TABLE 10.

**TABLE 10**

| Centrifuge-resuspension test | | | | |
|---|---|---|---|---|
| | Batch E | Batch F | Batch G | Batch H |
| Benzyl alcohol | 0% | 1% | 2% | 3% |
| Sedimentation | 92% | 84% | 78% | 79% |
| Resuspension time | 60 seconds | 28 seconds | 29 seconds | 25 seconds |

Syringeability and injectability tests were also conducted. 2 mL of sample was drawn from an inverted injection vial through an 18 gauge needle into a 3 mL syringe. The contents of the syringe were then discharged. Syringeability and injectability were acceptable, as indicated by TABLE 11.

**TABLE 11**

| Syringeability and injectability tests | | | | |
|---|---|---|---|---|
| | Batch E | Batch F | Batch G | Batch H |
| Benzyl alcohol | 0% | 1% | 2% | 3% |
| Syringeability time | 9 seconds | 8 seconds | 8 seconds | 9 seconds |
| Injectability time | 3 seconds | 3 seconds | 3 seconds | 3 seconds |

To summarize, sedimentation is faster when no benzyl alcohol is included in the formulations according to the present invention. The sedimentation volume is also smaller when no benzyl alcohol is included in the formulations according to the present invention. Centrifuge and resuspension testing shows resuspension time was quicker in formulations with benzyl alcohol. There was no significant difference in viscosity value with the addition of benzyl alcohol to the formulation. Syringeability and injectability of all formulations were acceptable.

Ceftiofur Hydrochloride Oily Suspension is a flocculated suspension. A flocculated suspension includes, but is not limited to, the following characteristics: 1) particles in the suspension are in the form of loose agglomerates, 2) the sediment is formed relatively rapidly, 3) the sediment is loosely packed, particles are not bound to each other tightly, 4) a hard cake is not formed, 5) the sediment is easily redispersed by a small amount of agitation, and 6) pressure distribution and viscosity are consistent throughout the depth of the product.

From the test data generated on the above formulation with and without Benzyl alcohol, it is observed that Benzyl alcohol has a surprising and unexpected effect on the flocculation. Benzyl alcohol reduces the rate of sedimentation and improves the rate of resuspension.

### EXAMPLE 3 (not according to the invention)

### Formulation development of a combination ceftiofur HCl and ketoprofen oily suspension

The following summarizes the development of a stable, easily resuspendable combination ceftiofur HCl and ketoprofen oily suspension for injection, containing 5.0 % w/v ceftiofur and 15.0% w/v ketoprofen suitable for intramuscular and subcutaneous injection. The desired formulation comprises 5.0% ceftiofur HCL and the excipients; PHOSPHOLIPON 90H, sorbitan monooleate, propylene glycol and benzyl alcohol in a refined cottonseed oil vehicle. Attempts were made to incorporate ethanol as an excipient into the original ceftiofur HCL injection formulation.

### Formulation 1

Purpose: Comparing the addition of various concentrations of benzyl alcohol and ethanol. NB - no ketoprofen.

Sub-batches had the following amounts of benzyl alcohol and ethanol:
1: 15% benzyl alcohol / 0% ethanol
2: 10% benzyl alcohol / 0% ethanol
3: 0% benzyl alcohol / 0% ethanol
4: 5% benzyl alcohol / 10% ethanol

Method: 170mL of ceftiofur HCL suspension was prepared according to TABLE 12. Appropriate amounts of benzyl alcohol, ethanol and/or cottonseed oil totaling 7.5 mL were added to 42.5 mL of the suspension. Component amounts are summarized in TABLE 12.

**TABLE 12**

| **Component** | **%w/v** |
|---|---|
| Ceftiofur (as Ceftiofur HCl) | 5.0 |
| Lecithin (PHOSPHOLIPON 90H) | 0.05 |
| Sorbitan monooleate (ARLACEL-80) | 0.15 |
| Propylene glycol | 0.25 |
| Refined cottonseed oil | to 100 |
| Benzyl alcohol | 0, 5, 10 or 15% |
| Ethanol | 0 or 10% |

Result: Sub-batches 1, 2 and 3 were well dispersed. Sub-batch 4 was not well dispersed, as indicated by deposits on the bottle wall surface.

### Formulation 2

Incorporation of ketoprofen into a formulation based on the original formulation with ethanol was then attempted. Formulation 2 components are summarized in TABLE 13.

Purpose: Ceftiofur HCl injection without ketoprofen.

Method: Heated cottonseed oil to 95-100°C, added lecithin and stirred until clear (40 minutes), cooled to 30°C. Next, added sorbitan monooleate and stirred for 10 minutes. Then added ceftiofur HCl in portions and stirred until completely dissolved. Continued stirring until completely dispersed (20 minutes). Finally added propylene glycol, benzyl alcohol and ethanol and stirred for about 10 minutes. Volume was brought to 100 using refined cottonseed oil. Component amounts are summarized in TABLE 13.

**TABLE 13**

| **Component** | **%w/v** |
|---|---|
| Ceftiofur (as Ceftiofur HCl) | 5.0 |
| Lecithin (PHOSPHOLIPON 90H) | 0.05 |
| Sorbitan monooleate (ARLACEL-80) | 0.15 |
| Propylene glycol | 0.25 |
| Benzyl alcohol | 10.0 |
| Ethanol | 5.0 |
| Refined cottonseed oil | to 100 |

Result: Formulations containing both active ingredients and employing the refined cottonseed oil as vehicle tended to result in a fairly viscous suspension.

### Formulation 3

Purpose: Ceftiofur HCl injection with ketoprofen

Method: Formulation 3 was prepared according to the method of Formulation 2, but with the addition of ketoprofen at the same time as the ceftiofur HCl. Component amounts are summarized in TABLE 14.

**TABLE 14**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate (ARLACEL-80) | 0.15 |
| Propylene glycol | 0.25 |
| Benzyl alcohol | 10.0 |
| Ethanol | 5.0 |
| Refined cottonseed oil | to 100 |

Result: Formulation 3 was viscous.

### Formulation 4

Purpose: Reduce benzyl alcohol to 1.0% and reduce ethanol to 0.0% to determine if removal of ethanol would resolve the problem of the high viscosity and "stickiness" of Formulation 3.

Sub-batches had the following amounts of ketoprofen:
1 - 0.0% ketoprofen
2 - 15.0% ketoprofen

Method: Formulation 4 was prepared according to the method of Formulation 3, but with either ceftiofur HCl only or ceftiofur HCl and ketoprofen added after the sorbitan monooleate. Component amounts are summarized in TABLE 15.

**TABLE 15**

| **Component** | **%w/v** |
|---|---|
| Ceftiofur (as Ceftiofur HCl) | 5.0 |
| Ketoprofen | 0 |
| Lecithin (PHOSPHOLIPON 90H) | 0.05 |
| Sorbitan monooleate (ARLACEL-80) | 0.15 |
| Propylene glycol | 0.25 |
| Benzyl alcohol | 1.0 |
| Refined cottonseed oil | to 100 |

Result: Formulation 4 was thick.

Based on these results, investigation of alternative oily vehicles suitable for the combination was commenced. Alternative vehicles trials included medium chain triglyceride oils (MIGLYOL), ethyl oleate, isopropyl myristate and glyceryl tricoprylate coprate (CRODAMOL GTCC), as well as 1:1 combinations of some of these vehicles.

### Formulation 5

Purpose: To test 10 % benzyl alcohol / 5 % ethanol formulation in medium chain triglyceride (MIGLYOL 810).

Method: Formulation 5 was prepared according to the method of Formulation 3, but cottonseed oil was replaced with MIGLYOL 810. Component amounts are summarized in TABLE 16.

**TABLE 16**

| **Component** | **%w/v** |
|---|---|
| Ceftiofur (as Ceftiofur HCl) | 5.0 |
| Ketoprofen | 15 |
| Lecithin (PHOSPHOLIPON 90H) | 0.05 |
| Sorbitan monooleate (ARLACEL-80) | 0.15 |
| Propylene glycol | 0.25 |
| Benzyl alcohol | 10.0 |
| Ethanol | 5.0 |
| MIGLYOL 810 | to 100 |

Result: MIGLYOL 810 had been added to try to reduce the viscosity of previous formulations. Formulation 5 was a good suspension.

### Formulation 6

Purpose: 1 % benzyl alcohol / 0 % ethanol formulation in medium chain triglyceride, MIGLYOL 810.

Sub-batch 1 - without ketoprofen (TABLE 17), sub-batch 2 - with ketoprofen (TABLE 18) Method: Formulation 6 was prepared according to the method of Formulation 4 with MIGLYOL 810 instead of cottonseed oil and either 0.0% or 15% ketoprofen. Component amounts are summarized in TABLE 17 (sub-batch 1) and TABLE 18 (sub-batch 2).

**TABLE 17**

| **Component** | **%w/v** |
|---|---|
| Ceftiofur (as Ceftiofur HCl) | 5.0 |
| Ketoprofen | 0 |
| Lecithin (PHOSPHOLIPON 90H) | 0.05 |
| Sorbitan monooleate (ARLACEL-80) | 0.15 |
| Propylene glycol | 0.25 |
| Benzyl alcohol | 1.0 |
| MIGLYOL 810 | to 100 |

**TABLE 18**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate (ARLACEL-80) | 0.15 |
| Propylene glycol | 0.25 |
| Benzyl alcohol | 1.0 |
| MIGLYOL 810 | to 100 |

Result: MIGLYOL 810 had been added to try to reduce the viscosity of previous formulations. Sub-batch 1 (no ketoprofen, TABLE 17) was a good suspension, whereas sub-batch 2 (15% ketoprofen, TABLE 18) was a very viscous, nearly solidified, suspension.

### Formulation 7

Purpose: 10 % benzyl alcohol / 5 % ethanol formulation in ethyl oleate and cottonseed oil vehicle

Method: Formulation 7 was prepared according to the method of Formulation 2, with Ethyl oleate and some cottonseed oil were heated to 95 -100°C in the first step. Component amounts are summarized in TABLE 19.

**TABLE 19**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate (ARLACEL-80) | 0.15 |
| Propylene glycol | 0.25 |
| Benzyl alcohol | 10.0 |
| Ethanol | 5.0 |
| Ethyl oleate | 30.0 |
| Refined cottonseed oil | to 100 |

Result: Formulation 7 was a good suspension.

### Formulation 8

Purpose: Prepare batch with 10% benzyl alcohol, 5% ethanol and cottonseed oil for stability.

Method: Formulation 8 was prepared according to the following steps: 1) Filtered refined cottonseed oil, 2) heated oil to 95 °C - 100 °C, 3) added lecithin with constant stirring until a clear solution developed, 4) cooled the oil/lecithin mixture to 30 °C, 5) added sorbitan monooleate and stirred continuously for 10 minutes, 6) added ceftiofur HCl and ketoprofen with continuous stirring for 15 minutes until completely dispersed, 7) added propylene glycol, benzyl alcohol and ethanol and continuously stirred for 10 minutes, 8) brought to volume with refined cotton seed oil, and 9) homogenized for 5 minutes. Component amounts are summarized in TABLE 20 and density measurements are summarized in TABLE 21.

**TABLE 20**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 10.0 |
| Ethanol | 5.0 |
| Refined cottonseed oil | to 100 |

Result: Density (20°C) was 0.9734 g/mL.

**TABLE 21**

| | **Active content (%LC)** | |
|---|---|---|
| | **Ceftiofur** | **Ketoprofen** |
| 4°C | 95.8 | 97.0 |
| 55°C / 2 weeks | 73.0 | 57.0 |
| 55°C / 4 weeks | 72.9 | 46.5 |

### Formulation 9

Purpose: Prepare batch with 10% benzyl alcohol, 5% ethanol and MIGLYOL 810 for stability.

Method: Formulation 9 was prepared as per the 10% benzyl alcohol, 5% ethanol and cottonseed oil batches, but the cottonseed oil was replaced with MIGLYOL 810 (NB oil not filtered prior to heating). Component amounts are summarized in TABLE 22 and density measurements are summarized in TABLE 23.

**TABLE 22**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 10.0 |
| Ethanol | 5.0 |
| MIGLYOL 810 | to 100 |

Result: Density (20°C) was 0.9951 g/mL.

**TABLE 23**

| | **Active content (%LC)** | |
|---|---|---|
| | **Ceftiofur** | **Ketoprofen** |
| 4°C | 75.4 | 76.3 |
| 55°C / 2 weeks | 73.2 | 59.9 |
| 55°C / 4 weeks | 88.5 | 61.0 |

### Formulation 10

Purpose: Prepare batch with 10% benzyl alcohol, 5% ethanol and Ethyl oleate / refined cottonseed oil for stability.

Method: Formulation 10 was prepared as per the 10% benzyl alcohol, 5% ethanol and cottonseed oil batches, but the cottonseed oil in step 2 was replaced with ethyl oleate and refined cottonseed oil (NB oil not filtered prior to heating). Component amounts are summarized in TABLE 24 and density measurements are summarized in TABLE 25.

**TABLE 24**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 10.0 |
| Ethanol | 5.0 |
| Ethyl oleate | 30.0 |
| Refined cottonseed oil | to 100 |

Result: Density (20 °C) was 0.9569 g/mL.

**TABLE 25**

| | **Active content (%LC)** | |
|---|---|---|
| | **Ceftiofur** | **Ketoprofen** |
| 4°C | 95.0 | 96.4 |
| 55°C / 2 weeks | 94.6 | 74.3 |
| 55°C / 4 weeks | 94.1 | 60.8 |

### Formulation 11

Purpose: Prepare batch with 1% benzyl alcohol, 0% ethanol and MIGLYOL 810 for physical stability.

Method: Formulation 11 was prepared as per the 10% benzyl alcohol, 5% ethanol and MIGLYOL 810 batches, but no ethanol was added. Component amounts are summarized in TABLE 26.

**TABLE 26**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| MIGLYOL 810 | to 100 |

Results: Resuspension was acceptable, sedimentation volume was 5.6 mL / 9.0 mL number of inversions to resuspend was less than 30.

### Formulation 12

Purpose: Prepare batch with 1% benzyl alcohol, 0% ethanol and Ethyl oleate for physical stability.

Method: Formulation 12 was prepared as per the 1% benzyl alcohol, 0% ethanol and MIGLYOL 810 batches, but MIGLYOL 810 was replaced with ethyl oleate. Component amounts are summarized in TABLE 27.

**TABLE 27**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| Ethyl oleate | to 100 |

### Formulation 13

Purpose: Prepare batch with 1% benzyl alcohol, 0% ethanol and ethyl oleate/ cottonseed oil (1:1) for physical stability.

Method: Formulation 13 was prepared as per the 1% benzyl alcohol, 0% ethanol and MIGLYOL 810 batches, but MIGLYOL 810 was replaced with ethyl oleate/refined cottonseed oil (1:1 v/v). Component amounts are summarized in TABLE 28.

**TABLE 28**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| Ethyl oleate / Refined cottonseed oil (1:1 v/v) | to 100 |

### Formulation 14

Purpose: Prepare batch with 1% benzyl alcohol, 0% ethanol and ethyl oleate/ MIGLYOL 810 (1:1) for physical stability.

Method: Formulation 14 was prepared as per the 1% benzyl alcohol, 0% ethanol and MIGLYOL 810 batches, but MIGLYOL 810 was replaced with ethyl oleate / MIGLYOL 810 (1:1 v/v). Component amounts are summarized in TABLE 29.

**TABLE 29**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| Ethyl oleate / MIGLYOL 810 (1:1 v/v) | to 100 |

### Formulation 15

Purpose: Prepare batch with 1% benzyl alcohol, 0% ethanol and MIGLYOL 810 for physical stability and stress testing.

Method: Formulation 15 was prepared as per the 1% benzyl alcohol, 0% ethanol and MIGLYOL 810 batches. Component amounts are summarized in TABLE 30.

**TABLE 30**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| MIGLYOL 810 | to 100 |

Result: The resuspension of Formulation 15 was acceptable, the sedimentation volume was 6.9 mL / 9.0 mL, and the number of inversions to resuspend was less than 30. Data are summarized in TABLE 31.

**TABLE 31**

| | **Active content (%LC)** | |
|---|---|---|
| | **Ceftiofur** | **Ketoprofen** |
| 4°C | 100.4 | 100.4 |
| Room temp. | 98.8 | 103.2 |
| 55°C / 2 weeks | 101.2 | 103.8 |
| 55°C / 4 weeks | 99.3 | 100.5 |

### Formulation 16

Purpose: Prepare batch with 1% benzyl alcohol, 0% ethanol and MIGLYOL 810 for chemical stability.

Method: Formulation 16 was prepared as per the 1% benzyl alcohol, 0% ethanol and MIGLYOL 810 batches. Component amounts are summarized in TABLE 32.

**TABLE 32**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| MIGLYOL 810 | to 100 |

Result: Resuspension of Formulation 16 was acceptable, sedimentation volume was 6.9 mL / 9.0 mL, and the number of inversions to resuspend was less than 30. Data are summarized in TABLE 33.

**TABLE 33**

| | **Active content (%LC)** | |
|---|---|---|
| | **Ceftiofur** | **Ketoprofen** |
| 4°C | 100.8 | 101.9 |
| Room temp. | 100.5 | 102.2 |
| 55°C / 2 weeks | 100.5 | 103.6 |
| 55°C / 4 weeks | 99.3 | 100.9 |

### Formulation 17

Purpose: Prepare batch with 1% benzyl alcohol, 0% ethanol and MIGLYOL 810 for chemical stability.

Method: Formulation 17 was prepared as per the 1% benzyl alcohol, 0% ethanol and MIGLYOL 810 batches. Component amounts are summarized in TABLE 34

**TABLE 34**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| MIGLYOL 810 | to 100 |

Result: Resuspension of Formulation 17 was acceptable, the sedimentation volume was 6.8 mL / 9.0 mL, and the number of inversions to resuspend was less than 30. Data are summarized in TABLE 35.

**TABLE 35**

| | **Active content (%LC)** | |
|---|---|---|
| | **Ceftiofur** | **Ketoprofen** |
| 4°C | 95.6 | 96.2 |
| Room temp. | 97.1 | 98.4 |
| 55°C / 2 weeks | 100.3 | 99.7 |
| 55°C / 4 weeks | 99.9 | 97.5 |

### Formulation 18

Purpose: Prepare batch with 1% benzyl alcohol, using IPM as oil vehicle.

Method: Formulation 18 was prepared as per the 1% benzyl alcohol, 0% ethanol and MIGLYOL 810 batches, with an additional first step wherein the MIGLYOL 810 was filtered through a 0.2µm filter. Component amounts are summarized in TABLE 36.

**TABLE 36**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| IPM | to 100 |

Result: Formulation 18 was not well-suspended; the sedimentation volume was 3.7 mL / 9.0 mL, and the number of inversions to resuspend was greater than 300.

### Formulation 19

Purpose: Prepare batch with 1% benzyl alcohol, using IPM / refined cottonseed oil (1:1 v/v) as oil vehicle.

Method: Formulation 19 was prepared as per the 1% benzyl alcohol, 0% ethanol and MIGLYOL 810 batches, with an additional first step wherein the MIGLYOL 810 was filtered through a 0.2µm filter. Component amounts are summarized in TABLE 37.

**TABLE 37**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| IPM / Refined cottonseed oil (1:1 v/v) | to 100 |

Result: Formulation 19 was not well-suspended; the sedimentation volume was 4.1 mL / 9.0 mL, and the number of inversions to resuspend was greater than 300.

### Formulation 20

Purpose: Prepare batch with 1% benzyl alcohol, using ethyl oleate / MIGLYOL 810 (1:1 v/v) as oil vehicle.

Method: Formulation 20 was prepared as per the 1% benzyl alcohol, 0% ethanol and MIGLYOL 810 batches, with an additional first step wherein the MIGLYOL 810 was filtered through a 0.2µm filter. Component amounts are summarized in TABLE 38 and the results of the stress study are summarized in TABLE 39.

**TABLE 38**

| **Component** | **%w/v** |
|---|---|
| Ceftiofur (as Ceftiofur HCl) | 5.0 |
| Ketoprofen | 15.0 |
| Lecithin (PHOSPHOLIPON 90H) | 0.05 |
| Sorbitan monooleate | 0.15 |
| Propylene glycol | 0.25 |
| Benzyl alcohol | 1.0 |
| Ethyl oleate / MIGLYOL 810 (1:1 v/v) | to 100 |

Result: Density of Formulation 20 was 0.972 g / mL.

**TABLE 39**

| | **Actives (% label claim)** | |
|---|---|---|
| | **Ceftiofur** | **Ketoprofen** |
| 4°C | 98.9 | 99.5 |
| 55°C / 2 weeks | 98 | 98.4 |
| 55°C / 4 weeks | 99.6 | 97.8 |

### Formulation 21

Purpose: Trial batch using CRODAMOL GTCC as oil vehicle.

Method: Formulation 21 was prepared as per the method of the trial batch with refined cottonseed oil, benzyl alcohol added before heating, but using CRODAMOL GTCC in place of cottonseed oil. Components are summarized in TABLE 40.

**TABLE 40**

| **Component** | **%w/v** |
|---|---|
| Ceftiofur HCl | 5.35 |
| Ketoprofen | 15.0 |
| PHOSPHOLIPON 90H | 0.05 |
| Sorbitan monooleate (CRILL 4) | 0.15 |
| Propylene glycol | 0.25 |
| Benzyl alcohol | 1.0 |
| CRODAMOL GTCC | to 100 |

Result: Formulation 21 was solid after overnight storage at 2-8°C, on shaking begins to move but not mobile. Viscosity (Ford cup No.4) was 38 seconds and the sedimentation after 1000 rpm for 15 minutes (8 mL) was 98% sediment. Formulation 21 resuspended in 10 seconds. The number of inversions to resuspend was 1.

Method: A 7-day separation was then performed on Formulation 21. The results are summarized

**TABLE 41**

| | **Clear** |
|---|---|
| Day 1 | 1% |
| Day 4 | 3% |
| Day 6 | 4% |
| Day 7 | 5% |

### Formulation 22

Purpose: Trial batch using ethyl oleate as oil vehicle.

Method: Formulation 22 was prepared as per the refined cottonseed oil batch with the these modifications: benzyl alcohol was added before heating, but ethyl oleate was used in place of cottonseed oil. Components are summarized in TABLE 42.

**TABLE 42**

| **Component** | **%w/v** |
|---|---|
| Ceftiofur HCl | 5.35 |
| Ketoprofen | 15.0 |
| PHOSPHOLIPON 90H | 0.05 |
| Sorbitan monooleate (CRILL 4) | 0.15 |
| Propylene glycol | 0.25 |
| Benzyl alcohol | 1.0 |
| Ethyl oleate | to 100 |

Result: Formulation 22 resuspended easily. The formulation was very viscous (but mobile) when stored at 2-8°C. The viscosity (Brookfield spindle 2 @ 100 rpm) was 102 mPas (cP) and the viscosity (Ford cup No.4) was 17 seconds. The sedimentation after 1000 rpm for 15 minutes (8 mL) was 79% sediment, and the time to resuspend was 30 seconds. The seven day separation data are presented in TABLE 43.

**TABLE 43**

| | **Sediment** | **Clear** |
|---|---|---|
| Day 1 | 95% | 5% |
| Day 2 | 95% | 5% |
| Day 5 | 92% | 8% |
| Day 7 | 92% | 8% |

This work provided indications that MIGLYOL 810 may have potential as a replacement for cottonseed oil. Further investigation into the lower viscosity MIGLYOL 840 resulted in MIGLYOL 840 becoming a preferred oil for further formulation development. Even though MIGLYOL 840 is preferred, MIGLYOL 810 is still contemplated as a potential replacement for cottonseed oil in formulations described herein.

### Formulation 23

Purpose: Trial batch using MIGLYOL 840 as oil vehicle.

Method: Formulation 23 was prepared as per the refined cottonseed oil batch with minor modifications: benzyl alcohol added before heating, MIGLYOL 840 was used in place of cottonseed oil. Components are summarized in TABLE 44.

**TABLE 44**

| **Component** | **%w/v** |
|---|---|
| Ceftiofur HCl | 5.35 |
| Ketoprofen | 15.0 |
| PHOSPHOLIPON 90H | 0.05 |
| Sorbitan monooleate (CRILL 4) | 0.15 |
| Propylene glycol | 0.25 |
| Benzyl alcohol | 1.0 |
| MIGLYOL 840 | to 100 |

Results: Formulation 23 was solid after overnight storage at 2-8°C, and on shaking began to move with moderate mobility. Viscosity was 18 seconds; sedimentation after 1000 rpm for 15 minutes (8 mL) was 90% sediment; resuspension occurred within 5 seconds; and the number of inversions to resuspend was 3. The seven day separation data is summarized in TABLE 45 and the stress study data is summarized in TABLE 46.

**TABLE 45**

| | **Clear** |
|---|---|
| Day 1 | 5 % |
| Day 4 | 5% |
| Day 6 | 8% |
| Day 7 | 8% |

**TABLE 46**

| | **Active Content** | | | |
|---|---|---|---|---|
| | **Ceftiofur** | | **Ketoprofen** | |
| | % | Recovery relative to 4°C (%) | % | Recovery relative to 4°C (%) |
| 4°C | 4.985 | | 14.53 | |
| 55C/4wk | 4.951 | 99.3 | 13.11 | 90.2 |

Further work was carried out to investigate the effect of different levels of benzyl alcohol on formulations with the MIGLYOL 840 oil vehicle using either representative active concentrations of ceftiofur (5.0%) and ketoprofen (15.0%), or a representative lower active concentration of ceftiofur (3.57%) and ketoprofen (10.0%). In contrast to the effect benzyl alcohol had on formulations containing only ceftiofur in the refined cottonseed vehicle, the benzyl alcohol did not appear to effect the flocculation of the formulations containing both ceftiofur and ketoprofen.

During the investigation of the effect of benzyl alcohol, stress testing (up to four weeks storage at 55°C) of batches containing 0, 1.0, 3.0 and 5.0% benzyl alcohol was carried out. It was found from the stress testing that the assay values of the ceftiofur after four weeks at 55°C remained relatively constant regardless of the benzyl alcohol content, but that the assay values for the ketoprofen after storage at 55°C for this period of time were reduced with increasing concentration of benzyl alcohol.

### Formulation 24

Purpose: Compare batches (5% Ceftiofur / 15% Ketoprofen) in MIGLYOL 840 with: 0% benzyl alcohol (ANT0088-25), 1% benzyl alcohol (ANT0088-26), 3% benzyl alcohol (ANT0088-27), and 5% benzyl alcohol (ANT0088-28).

Method: As per batch with refined cottonseed oil - benzyl alcohol added before heating.

**TABLE 47**

| **Component** | **%w/v** |
|---|---|
| Ceftiofur HCl | 5.35 |
| Ketoprofen | 15.0 |
| PHOSPHOLIPON 90H | 0.05 |
| Sorbitan monooleate (CRILL 4) | 0.15 |
| Propylene glycol | 0.25 |
| Benzyl alcohol | 0 |
| MIGLYOL 840 | to 100 |
| Ceftiofur HCl | 5.35 |
| Ketoprofen | 15.0 |
| PHOSPHOLIPON 90H | 0.05 |
| Sorbitan monooleate (CRILL 4) | 0.15 |
| Propylene glycol | 0.25 |
| Benzyl alcohol | 1.0 |
| MIGLYOL 840 | to 100 |
| Ceftiofur HCl | 5.35 |
| Ketoprofen | 15.0 |
| PHOSPHOLIPON 90H | 0.05 |
| Sorbitan monooleate (CRILL 4) | 0.15 |
| Propylene glycol | 0.25 |
| Benzyl alcohol | 3.0 |
| MIGLYOL 840 | to 100 |
| Ceftiofur HCl | 5.35 |
| Ketoprofen | 15.0 |
| PHOSPHOLIPON 90H | 0.05 |
| Sorbitan monooleate (CRILL 4) | 0.15 |
| Propylene glycol | 0.25 |
| Benzyl alcohol | 5.0 |
| MIGLYOL 840 | to 100 |

Result: All 4 batches were solid after 24 and 48 hours storage at 2-8°C, but became mobile on shaking.

**TABLE 48**

| | **25** | **26** | **27** | **28** |
|---|---|---|---|---|
| % BA | 0 | 1.0 | 3.0 | 5.0 |

| ***Viscosity (Brookfield, spindle 2, 100rpm) mPas (cP)*** | | | | |
|---|---|---|---|---|
| | 158 | 162 | 142 | 144 |

| ***Centrifuge test 1000rpm, 15 mins:*** | | | | |
|---|---|---|---|---|
| % sediment | 88 | 90 | 89 | 92 |
| Re-suspn time (sec) | 27 | 29 | 28 | 28 |

| ***Syringeability (18 g needle) sec:*** | | | | |
|---|---|---|---|---|
| | 8 | 7 | 7 | 6 |

| ***Injectability, sec:*** | | | | |
|---|---|---|---|---|
| | 9 | 5 | 6 | 5 |

| ***Sedimentation Volume (%)*** | | | | |
|---|---|---|---|---|
| Day 0 | 100 | 100 | 100 | 100 |
| Day 1 | 99 | 99 | 99 | 99 |
| Day 2 | 99 | 98 | 98 | 98 |
| Day 3 | 99 | 98 | 98 | 97 |
| Day 4 | 99 | 98 | 98 | 96 |
| Day 5 | 99 | 97 | 97 | 96 |
| Day 6 | 99 | 97 | 97 | 95 |
| Day 7 | 99 | 96 | 96 | 94 |
| Day 8 | 99 | 95 | 95 | 93 |
| Day 9 | 99 | 95 | 95 | 93 |
| Day 30 | 95 | 90 | 90 | 86 |
| 6 month | 88 | 83 | 79 | 72 |

**TABLE 49**

| **Batch No. ANT008** - | **Condition** | **Active Content** | | | |
|---|---|---|---|---|---|
| | | **Ceftiofur** | | **Ketoprofen** | |
| | | % | Recovery relative to 4°C (%) | % | Recovery relative to 4°C (%) |
| **25** | 4°C | 5.182 | | 14.74 | |
| | 55/4wk | 5.026 | 97.0 | 14.21 | 96.4 |
| **26** | 4°C | 5.238 | | 14.81 | |
| | 55/4wk | 5.064 | 96.7 | 14.10 | 95.2 |
| **27** | 4°C | 5.129 | | 14.60 | |
| | 55/4wk | 4.990 | 97.3 | 13.54 | 92.7 |
| **28** | 4°C | 5.215 | | 14.79 | |
| | 55/4wk | 5.095 | 97.7 | 13.18 | 89.1 |

In order to optimize the long term stability of the formulation, a formulation trial batch was produced using 0.50%w/v chlorobutanol as preservative, rather than benzyl alcohol.

### Formulation 25

Purpose: To prepare a batch (1% benzyl alcohol, with MIGLYOL base) using 0.5% chlorobutanol as preservative in place of benzyl alcohol.

Method: As per batch with refined cottonseed oil - benzyl alcohol was added before heating, with no benzyl alcohol. Chlorobutanol was added to heated MIGLYOL 840.

**TABLE 50**

| **Component** | **%w/v** |
|---|---|
| Ceftiofur HCl | 5.35 |
| Ketoprofen | 15.0 |
| PHOSPHOLIPON 90H | 0.05 |
| Sorbitan monooleate (CRILL 4) | 0.15 |
| Propylene glycol | 0.25 |
| Chlorobutanol | 0.50 |
| MIGLYOL 840 | to 100 |

Result: Viscosity (Brookfield, spindle 2, 100 rpm) was 112 mPas (cP), the sedimentation after 1000 rpm for 15 minutes (8 mL) was 91% sediment.

Long-term sedimentation:

**TABLE 51**

| | **Sedimentation volume** |
|---|---|
| Day 0 | 100% |
| Day 1 | 99% |
| Day 2 | 98% |
| Day 3 | 97% |
| Day 4 | 96% |
| Day 7 | 95% |
| 6 month | 90% |

Development work was carried out with the aim of producing an effective, well-flocculated suspension which readily resuspends to produce a uniform suspension on shaking. A test involving centrifugation of samples at 1000 rpm for 15 minutes was performed on most of the formulation batches produced. Once samples were centrifuged, the percentage sedimentation produced and time for resuspension were tested and could be compared for the various formulation batches. Formulation 25 batches produced 90% sediment following centrifugation, and were subsequently resuspended in about 29 seconds. The sedimentation volume measured for sample batches also became 90% after standing for 30 days.

During use an effective suspension must also be withdrawn from a container and administered by syringe. Syringeability and injectability studies involving the withdrawal and discharge of a 2 mL sample using an 18 gauge needle and 3 mL syringes have been carried out on formulation batches containing 0 to 5.0% benzyl alcohol. Syringeability time using a plastic syringe ranged from six to eight seconds. The syringeability of the 1.0% benzyl alcohol formulation was 7 seconds which was considered acceptable.

In general, formulation batches of up to 400 mL have been produced during development to date. The manufacturing process used was the same as that developed for the ceftiofur-only injection formulation, with the addition of both actives to a mixture of MIGLYOL 840, preservative, PHOSPHOLIPON 90H and sorbitan monooleate once it had cooled to less than 30°C, followed by addition of propylene glycol and making to volume with the MIGLYOL 840 vehicle. This method is detailed in the schematic diagram of FIG. 1.

During formulation development, investigation of the physical characteristics of the suspensions produced tended to be carried out with the formulations stored in either 100 mL clear glass measuring cylinders or 100 mL glass vials (either clear or amber), or in many cases both. The proposed packaging format may be 100 mL clear, type I glass vials with stoppers.

As the finished product is an injectable product in a multi-use vial, it needs to be both sterile and capable of maintaining its sterility with multiple challenges. As an oily suspension the alternative techniques to produce a sterile product include either aseptic manufacture or gamma irradiation. Terminal sterilization by gamma irradiation has been previously investigated in the ceftiofur-only formulation. Investigation on the gamma irradiation of the final proposed ceftiofur HCl / ketoprofen formulation is pending.

Benzyl alcohol 1.0% w/v or chlorobutanol 0.50% w/v was included in the proposed formulation. At the concentrations indicated in TABLE 52, these agents are expected to act as an effective preservative against the multiple challenges of a multi-use dose presentation

The components of one preferred formulation is provided in TABLE 52.

**TABLE 52**

| Proposed Formulation | | |
|---|---|---|
| **Ingredient** | **w/v% Composition** | **Function** |
| Ceftiofur (as Ceftiofur hydrochloride) | 5.0 | Active |
| Ketoprofen | 15.0 | Active |
| PHOSPHOLIPON 90H | 0.05 | Wetting / dispersing agent |
| Chlorobutanol | 0.50 | Preservative |
| Sorbitan monooleate | 0.15 | Wetting / dispersing agent |
| Propylene glycol | 0.25 | Flocculating agent / resuspendability enhancer |
| MIGLYOL 840 | to 100% | Biocompatible oil vehicle |

Testing of this formulation demonstrated that it was highly stable at both high temperatures and for prolonged periods when stored at room temperature. These data are summarized in TABLE 53.

**TABLE 53**

| **Batch No.** | **Storage Condition** | **Assay of Ceftiofur (%)** | **Assay of Ketoprofen (%)** |
|---|---|---|---|
| ANT0088-30 (0.5% Chlorobutanol) | 2-8°C | 103.2 | 100.1 |
| | 55°C / 4 weeks | 99.8 | 99.3 |
| | Room Temp. 9 months | 101.2 | 98.3 |
| | Room Temp. 12 months | 97.2 | 98.1 |

## Claims

1. A veterinarily or pharmaceutically acceptable formulation comprising:
an injectable suspension of an effective amount of ceftiofur, or a pharmaceutically acceptable salt thereof;
a biocompatible oil vehicle;
a wetting or dispersing agent which comprises a lecithin which contains a minimum of 90.0% hydrogenated phosphatidylcholine, a maximum of 4.0% hydrogenated lysophosphatidylcholine, and a maximum of 2% oil or triglycerides; and
at least one flocculating agent or resuspendability enhancer, and wherein the at least one flocculating agent or resuspendability enhancer comprises at least benzyl alcohol;
wherein the benzyl alcohol is present in an amount of 1% to 5% w/v.

2. The formulation of claim 1 wherein the ceftiofur is ceftiofur HCl.

3. The formulation of claim 1 or claim 2 wherein the ceftiofur is present in an amount of 1% to 10% w/v.

4. The formulation of any one of claims 1 to 3 wherein the flocculating agent or resuspendability enhancer further comprises propylene glycol.

5. The formulation of any one of claims 1 to 4 wherein the biocompatible oil vehicle comprises cottonseed oil.

6. The formulation according to claim 2 comprising: (a) ceftiofur HCl, (b) benzyl alcohol (c) a lecithin which contains a minimum of 90.0% hydrogenated phosphatidylcholine, a maximum of 4.0% hydrogenated lysophosphatidylcholine, and a maximum of 2% oil or triglycerides, (d) sorbitan monooleate, (e) propylene glycol and (f) cottonseed oil.

7. The formulation of claim 6 wherein (a) the ceftiofur HCl is present in an amount of 5.35% w/v, (b) the benzyl alcohol is present in an amount of 1% to 3% w/v, (c) the lecithin is present in an amount of 0.05% w/v, (d) the sorbitan monooleate is present in an amount of 0.15% w/v, (e) the propylene glycol is present in an amount of 0.25% w/v and (f) the cottonseed oil is present in an amount of up to 94% w/v.

8. A formulation according to any one of claims 1 to 7, for use in preventing or treating a respiratory disorder or disease in an animal.

## Patentansprüche

1. Eine veterinärmedizinisch oder pharmazeutisch verträgliche Formulierung, umfassend:
eine injizierbare Suspension mit einer wirksamen Menge von Ceftiofur oder eines pharmazeutisch verträglichen Salzes davon;
ein biokompatibles Ölvehikel;
ein Benetzungs- oder Dispergiermittel, welches ein Lecithin umfasst, das mindestens 90,0% hydriertes Phosphatidylcholin, höchstens 4,0% hydriertes Lysophosphatidylcholin und höchstens 2% Öl oder Triglyceride enthält; und
mindestens ein Flockungsmittel oder Resuspendierbarkeits-Verstärkungsmittel und wobei das mindestens eine Flockungsmittel oder Resuspendierbarkeits-Verstärkungsmittel mindestens Benzylalkohol umfasst; wobei der Benzylalkohol in einer Menge von 1 % bis 5% w/v vorliegt.

2. Die Formulierung nach Anspruch 1, wobei es sich bei dem Ceftiofur um Ceftiofur HCl handelt.

3. Die Formulierung nach Anspruch 1 oder Anspruch 2, wobei das Ceftiofur in einer Menge von 1% bis 10% w/v vorliegt.

4. Die Formulierung nach einem der Ansprüche 1 bis 3, wobei das Flockungsmittel oder Resuspendierbarkeits-Verstärkungsmittel ferner Propylenglykol umfasst.

5. Die Formulierung nach einem der Ansprüche 1 bis 4, wobei das biokompatible Ölvehikel Baumwollsamenöl umfasst.

6. Die Formulierung nach Anspruch 2, umfassend: (a) Ceftiofur HCl, (b) Benzylalkohol, (c) ein Lecithin, welches mindestens 90,0% hydriertes Phosphatidylcholin, höchstens 4,0 % hydriertes Lysophosphatidylcholin und höchstens 2% Öl oder Triglyceride enthält, (d) Sorbitanmonooleat, (e) Propylenglykol und (f) Baumwollsamenöl.

7. Die Formulierung nach Anspruch 6, wobei (a) das Ceftiofur HCl in einer Menge von 5,35% w/v vorliegt, (b) das Benzylalkohol in einer Menge von 1% bis 3% w/v vorliegt, (c) das Lecithin in einer Menge von 0,05% w/v vorliegt, (d) das Sorbitanmonooleat in einer Menge von 0,15% w/v vorliegt, (e) das Propylenglykol in einer Menge von 0,25% w/v vorliegt und (f) das Baumwollsamenöl in einer Menge von bis zu 94% w/v vorliegt.

8. Eine Formulierung nach einem der Ansprüche 1 bis 7, zur Verwendung bei der Vorbeugung oder Behandlung einer Atemwegsstörung oder -erkrankung bei einem Tier.

## Revendications

1. Formulation acceptable du point de vue vétérinaire ou pharmaceutique comprenant:
une suspension injectable d'une quantité efficace de ceftiofur, ou d'un sel pharmaceutiquement acceptable de celui-ci;
un véhicule huileux biocompatible;
un agent mouillant ou dispersant qui comprend une lécithine qui contient un minimum de 90,0 % de phosphatidylcholine hydrogénée, un maximum de 4,0 % de lysophosphatidylcholine hydrogénée, et un maximum de 2 % d'huile ou de triglycérides; et
au moins un agent floculant ou un agent augmentant l'aptitude à la remise en suspension, et où le au moins un agent floculant ou agent augmentant l'aptitude à la remise en suspension comprend au moins de l'alcool benzylique;
où l'alcool benzylique est présent en une quantité de 1 % à 5 % p/v.

2. Formulation selon la revendication 1 où le ceftiofur est du ceftiofur HCl.

3. Formulation selon la revendication 1 ou la revendication 2 où le ceftiofur est présent en une quantité de 1 % à 10 % p/v.

4. Formulation selon l'une quelconque des revendications 1 à 3 où l'agent floculant ou agent augmentant l'aptitude à la remise en suspension comprend en outre du propylèneglycol.

5. Formulation selon l'une quelconque des revendications 1 à 4 où le véhicule huileux biocompatible comprend de l'huile de coton.

6. Formulation selon la revendication 2 comprenant: (a) du ceftiofur HCl, (b) de l'alcool benzylique, (c) une lécithine qui contient un minimum de 90,0 % de phosphatidylcholine hydrogénée, un maximum de 4,0 % de lysophosphatidylcholine hydrogénée, et un maximum de 2 % d'huile ou de triglycérides; (d) du monooléate de sorbitan, (e) du propylèneglycol et (f) de l'huile de coton.

7. Formulation selon la revendication 6 où (a) le ceftiofur HCl est présent en une quantité de 5,35 % p/v, (b) l'alcool benzylique est présent en une quantité de 1 % à 3 % p/v, (c) la lécithine est présente en une quantité de 0,05 % p/v, (d) le monooléate de sorbitan est présent en une quantité de 0,15 % p/v, (e) le propylèneglycol est présent en une quantité de 0,25 % p/v et (f) l'huile de coton est présente en une quantité de jusqu'à 94 % p/v.

8. Formulation selon l'une quelconque des revendications 1 à 7, destinée à être utilisée dans la prévention ou le traitement d'un trouble ou maladie respiratoire chez un animal.
